(19) Europäisches Patentamt
European Patent Office
Office européen des brevets

(11) EP 1 682 194 B2

(12) # NEUE EUROPÄISCHE PATENTSCHRIFT

Nach dem Einspruchsverfahren

(45) Veröffentlichungstag und Bekanntmachung des Hinweises auf die Entscheidung über den Einspruch:
**29.03.2017 Patentblatt 2017/13**

(45) Hinweis auf die Patenterteilung:
**09.07.2008 Patentblatt 2008/28**

(21) Anmeldenummer: **04790951.0**

(22) Anmeldetag: **28.10.2004**

(51) Int Cl.:
***A61L 15/48*** *(2006.01)*

(86) Internationale Anmeldenummer:
**PCT/EP2004/012178**

(87) Internationale Veröffentlichungsnummer:
**WO 2005/042042 (12.05.2005 Gazette 2005/19)**

(54) **BLUT UND/ODER KÖRPERFLÜSSIGKEITEN ABSORBIERENDE POLYMERPARTIKEL**

BLOOD- AND/OR BODY FLUID-ABSORBING POLYMER PARTICLES

PARTICULES POLYMERES ABSORBANT LE SANG ET/OU DES LIQUIDES ORGANIQUES

(84) Benannte Vertragsstaaten:
**AT BE BG CH CY CZ DE DK EE ES FI FR GB GR HU IE IT LI LU MC NL PL PT RO SE SI SK TR**

(30) Priorität: **31.10.2003 DE 10351267**
**28.06.2004 DE 102004035671**

(43) Veröffentlichungstag der Anmeldung:
**26.07.2006 Patentblatt 2006/30**

(73) Patentinhaber: **BASF SE**
**67056 Ludwigshafen am Rhein (DE)**

(72) Erfinder:
- **ZIEMER, Antje**
  **68199 Mannheim (DE)**
- **STEINMETZ, Bernhard**
  **97535 Rütschenhausen (DE)**
- **DE MARCO, Michael**
  **69469 Weinheim (DE)**
- **MEDELNICK, Monika**
  **67122 Altrip (DE)**

(74) Vertreter: **BASF IP Association**
**BASF SE**
**G-FLP-C006**
**67056 Ludwigshafen (DE)**

(56) Entgegenhaltungen:
**EP-A- 0 759 460** **DD-A1- 96 965**
**JP-A- S6 116 903** **JP-A- H02 153 903**

- **PATENT ABSTRACTS OF JAPAN Bd. 1995, Nr. 03, 28. April 1995 (1995-04-28) & JP 06 345980 A (SANYO CHEM IND LTD), 20. Dezember 1994 (1994-12-20) in der Anmeldung erwähnt**

EP 1 682 194 B2

## Beschreibung

Die vorliegende Erfindung betrifft Blut und/oder Körperflüssigkeiten absorbierende Polymerpartikel sowie deren Anwendung zur Absorption von Blut und/oder Körperflüssigkeiten, insbesondere in Hygieneartikeln.

Flüssigkeiten absorbierende Polymere, sogenannte Superabsorber (Super-Absorbing Polymers, SAP), sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylehoxid oder in wässrigen Flüssigkeiten quellbare Naturprodukte, wie beispielsweise Guarderivate. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau oder zur Verdickung aller Arten von Abfällen, insbesondere von medizinischen Abfällen, verwendet.

Flüssigkeiten absorbierende Polymere sind bevorzugt solche mit einer Absorption von 0,9 Gew.-%iger Kochsalzlösung von mindestens dem 10-fachen Eigengewicht bezogen auf das eingesetzte Polymer, bevorzugt dem 20-fachen Eigengewicht. Diese Absorption wird bevorzugt auch unter einem Druck beispielsweise von 4830 Pa (0,7 psi) erreicht.

Zur Verbesserung der Anwendungseigerischaften werden Flüssigkeiten absorbierende Polymere üblicherweise oberflächen- oder gelnachvernetzt.

Diese Nachvernetzung ist dem Fachmann an sich bekannt und erfolgt bevorzugt in wäßriger Gelphase oder als Oberflächennachvernetzung der gemahlenen und abgesiebten Polymerpartikel.

Herkömmliche Superabsorber sind daraufhin optimiert, in Hygieneartikeln, vor allem Babywindeln, Urin zu absorbieren. Sie zeigen immer eine wesentlich geringere Absorption von Blut im Vergleich zu synthetischem Urin oder physiologischer Kochsalzlösung.

Es ist daher vorteilhaft einen Superabsorber bereitzustellen, der in der Lage ist, größere Mengen Blut als handelsübliche Superabsorber zu absorbieren. Derartige Superabsorber können bevorzugt in Produkten der Damenhygiene eingesetzt werden.

Bei Produkten der Damenhygiene ist eine rasche Blutabsorption wünschenswert, um die Flüssigkeit rasch vom Körper weg zu transportieren und in dem Hygieneartikel zu speichern. Neben der raschen Absorption und hoben Quellgeschwindigkeit ist auch eine hohe Retention notwendig.

Für Hygieneartikel ist die höhere Blutabsorption ein wesentlicher Vorteil, da dadurch die Entwicklung von sehr effektiven und dünnen Hygieneartikeln möglich ist, die vom Kunden aufgrund des Tragekomforts bevorzugt werden.

Die Patentanmeldung WO-A-99/55767 beschreibt die Verwendung von Aluminaten zur Oberflächennachvernetzung von unvernetzten oder kovalent vernetzte Hydrogelen. Die Anmeldung lehrt, dass durch die nachträgliche Vernetzung die Gelstärke, die Absorption von Flüssigkeiten und Blut, insbesondere die Absorption unter Druck, verbessert wird.

Die Patentanmeldung DE-A-199 09 653 lehrt das Aufbringen einer wässrigen Lösung eines Kations vor oder nach einer Nachvernetzungsreaktion.

Die Patentanmeldung WO-A-00/10496 beschreibt ein optimiertes Material zur Absorption von Blut durch Aufbringen von Kaolinit auf befeuchteten Superabsorber und anschließende Trocknung.

Die Patentanmeldung EP-A-0 759 460 beschreibt ein Material, welches durch Zusatz von großen Mengen eines Oberflächennachvernetzungsreagenzes erneut nachvernetzt wurde. Die höhere Vernetzung führt allerdings nicht zu Produkten mit sehr hoher Blutabsorption.

Die Patentanmeldung WO-A-95/19191 beschreibt die Herstellung von superabsorbierenden Materialien mit verbesserter Blutabsorption. Dazu werden handelsübliche Superabsorber zusätzlich mit Polyolen, wie beispielsweise Polyethylenglykol oder Glycerin besprüht. Die zusätzliche Vernetzung der Polymerstränge erfolgt dabei im wesentlichen durch Wasserstoffbrückenbindungen. Wie die Beispiele in der Anmeldung zeigen, läßt sich die Blutabsorption durch eine höhere Aufwandmenge nicht weiter steigern. Vielmehr nimmt bei höherer Dosierung die Blutbsorption wieder ab.

Die Patentanmeldung JP-A-06/345980 beschreibt die Mischung von Superabsorbern mit anionischen Tensiden. Dies verbessert weder die Blutabsorption, noch erhöht es die Absorptionsgeschwindigkeit.

Es bestand daher die Aufgabe, einen Superabsorber mit verbesserter Blutabsorption zu entwickeln.

Die Aufgabe wurde gelöst durch die Bereitstellung absorbierender Polymerpartikel, enthaltend

a) mindestens ein einpolymerisiertes ethylenisch ungesättigtes, säuregruppentragendes Monomer,
b) mindestens einen einpolylmerisierten Vernetzer,
c) gegebenenfalls ein oder mehrere mit a) copolymerisierbare einpolymerisierte ethylenisch und/oder allylisch ungesättigte Monomere,
d) gegebenenfalls ein oder mehrere wasserlösliche Polymere, auf die die Monomere a), b) und ggf. c) zumindests teilweise aufgepfropft sind, und

e) gegebenenfalls ein oder mehrere umgesetzte Nachvernetzer,

wobei die Polymerpartikel mit mindestens einem Tensid und mindestens einem Lösungsmittel der allgemeinen Formel I

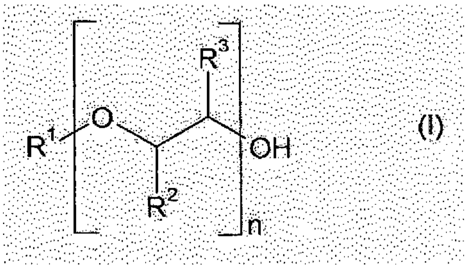

(I)

in der

$R^1$ $C_1$-$C_8$-Alkyl, wobei der Rest halogensubstituiert sein kann,

$R^2$, $R^3$ unabhängig voneinander Wasserstoff oder Methyl und

n eine ganze Zahl von 0 bis 5

bedeuten,
beschichtet sind und die trockenen Polymerpartikel eine Blutabsorption von mindestens 15 g/g aufweisen.

Das wenigstens eine Tensid kann ein anionisches, kationisches und/oder nichtionisches Tensid sein. Nichtionische Tenside sind bevorzugt, insbesondere nichtionische Tenside mit einem HLB-Wert von 2 bis 18. Der HLS-Wert ist ein Maß für die Wasser-bzw. Ol-Löslichkeit von vorwiegend nichtionischen Tensiden und kann nach üblichen Methoden bestimmt werden.

Ein Tensid besteht aus mindestens einer unpolaren und mindestens einer polaren Gruppe. Bevorzugte Tenside weisen große unpolare und/oder polare Gruppen auf. Große Gruppen sind Gruppen mit einem Molgewicht von mindestens 130 g/mol, vorzugsweise mindestens 250 g/mol, besonderes bevorzugt mindestens 500 g/mol.

Geeignete Tenside sind beispielsweise Sorbitanester, wie Sorbitanmonostearat, Sorbitanmonooleat, Sorbitanpalmitat und Sorbitallaurat sowie Glycerinester, deren Säurekomponente sich von $G_{14}$- bis $C_{20}$-Carbonsäuren ableitet.

Bevorzugte Tenside sind alkoxilierte, vorzugsweise ethoxilierte, $C_6$-$C_{20}$-Alkohole, wobei die Alkohole ggf. verzweigt und/oder ungesättigt sein können, sowie alkoxilierte, vorzugsweise ethoxilierte, Sorbitanmonoester, wie Sorbitanmonostearat und Sorbitanmonooleat.

Das wenigstens eine Tensid hat vorzugsweise eine Viskosität von über 20 mPas, besonders bevorzgt von über 25 mPas, ganz besonders bevorzugt von über 30 mPas (gemessen bei 23°C gemäß EN12092).

Bevorzugte Lösungsmittel sind Alkohol oder alkoxilierte Alkohole der allgemeinen Formel I, in der

$R^1$ $C_2$-$C_6$-Alkyl,

$R^2$, $R^3$ Wasserstoff und,

n eine ganze Zahl von 1 bis 3 bedeuten.

Ganz besonders bevorzugte Lösungsmittel sind alkoxilierte Alkohole der allgemeinen Formel I, in der

$R^1$ $C_3$-$C_5$-Alkyl,

$R^2$, $R^3$ Wasserstoff und

n 2 bedeutet.

Beispielsweise seien als Lösungsmittel genannt Ethylenglykolmonoethylether, Ethylenglykolmono-n-propylether, Ethylenglykolmonoisopropylether, Ethylenglykolmono-n-butylether, Ethylenglykolmono-sek.-butylether, Ethylenglykolmonoisobutylether, Ethylenglykolmono-tert.-butylether, Ethylenglykolmono-n-pentylether, Ethylenglykolmono-sek.-pentylether, Ethylenglykolmonoisopentylether, Ethylenglykolmono-tert.-pentylether, Ethylenglykolmono-n-hexylether, Diethylenglykolmonoethylether, Diethylenglykolmono-n-propylether, Diethylenglykolmonoisopropylether, Diethylenglykolmonon-butylether, Diethylenglykolmono-sek.-butylether, Diethylenglykolmonoisobutylether, Diethylenglykolmono-tert.-butylether, Diethylenglykolmono-n-pentylether, Diethylenglykolmano-sek.-pentylether, Diethylenglykolmonoisopentylether, Diethylenglykolmonotert.-pentylether, Diethylenglykolmono-n-hexylether, Triethylenglykolmonoethylether, Triethylenglykolmono-n-propylether, Triethylenglykolmonoisopröpylether, Triethylenglykolmono-n-butylether, Triethylenglykolmono-sek.-butylether, Triethylenglykolmonoisobutylether, Triethylenglykolmono-tert.-butylether, Triethylenglykolmono-n-pentylether, Triethylenglykolmono-sek.-pentylether, Triethylenglykolmonoisopentylether, Triethylenglykolmono-tert.-pentylether und Triethylenglykolmono-n-hexylether.

Das wenigstens eine Lösungsmittel hat vorzugsweise eine Viskosität von weniger als 20 mPas, besonders bevorzugt von weniger als 15 mPas, ganz besonders bevorzugt von weniger als 10 mPas (gemessen bei 23°C gemäß EN12092). Weitere Lösungsmittel können zusätzlich verwendet werden.

In einer bevorzugten Ausführungsform sind die absorbierenden Polymerpartikel zusätzlich mit mehrwertigen Metallkationen beschichtet, wobei Aluminiumkationen besonders bevorzugt sind.

Die erfindungsgemäßen Polymerpartikel weisen eine Blutabsorption, gemessen mit den trockenen Polymerpartikeln, von mindestens 15 g/g, vorzugsweise mindestens 20 g/g, bevorzugt mindestens 25 g/g, besonders bevorzugt mindestens 27 g/g, ganz besonders bevorzugt mindestens 29 g/g, auf.

Die erfindungsgemäßen absorbierenden Polymerpartikel können hergestellt werden durch eine Nachbehandlung von absorbierenden Polymeren umfassend die Schritte:

- Nachbehandlung mit wenigstens einem anionischen, kationischen und/oder nichtionischen Tensid, vorzugsweise einem nichtionischen Tensid mit einem HLB-Wert von 2 bis 18.

- Nachbehandlung mit wenigstens einem Lösungsmittel der allgemeinen Formel I, worin $R^1$, $R^2$, $R^3$ und n die obengenannten Bedeutungen haben, vorzugsweise Diethylenglykolmonobutylether.

Die beiden Verfahrensschritte werden üblicherweise gleichzeitig durchgeführt. Das Tensid wird dabei vorzugsweise in dem Lösungsmittel gelöst auf den getrockneten und klassierten Superabsorber aufgesprüht. Dadurch wird nicht nur das Tensid gleichmäßiger verteilt, sondern zusätzlich die Blutabsorption verbessert.

Die erfindungsgemäßen absorbierenden Polymerpartikel können hergestellt werden durch eine Nachbehandlung von absorbierenden Hydrogelen umfassend die Schritte:

- Nachbehandlung mit wenigstens einem mehrwertigen Metallkation, Lösungen mehrwertiger Metallkationen, wie beispielsweise $Al^{3+}$, $Fe^{2+}$, $Fe^{3+}$, $Ti^{3+}$, $Ti^{4+}$, $Co^{2+}$, $Ni^{2+}$, $Cr^{3+}$, $Mn^{2+}$, $Zn^{2+}$, $Mg^{2+}$, $Ca^{2+}$, $Zr^{3+}$, $Zr^{44}$, besonders bevorzugt $Al^{3+}$.

- Nachbehandlung mit wenigstens einem anionischen, kationischen und/oder nichtionischen Tensid, vorzugsweise einem nichtionischen Tensid mit einem HLB-Wert von 2 bis 18.

- Nachbehandlung mit wenigstens einem Lösungsmittel der allgemeinen Formel I, vorzugsweise Diethylenglykolmonobutylether.

Die beiden letzten Verfahrensschritte werden üblicherweise gleichzeitig durchgeführt.

Eingesetzt werden typischerweise 0,01 bis 5 Gew.-%, vorzugsweise 0,05 bis 4 Gew.-%, besonders bevorzugt 0,1 bis 3 Gew.-%, des Tensids, bezogen auf das absorbierende Polymer.

Die Menge an Lösungsmittel der allgemeinen Formel I beträgt üblicherweise-0,1 bis 10 Gew.-%, vorzugsweise 0,5 bis 7 Gew.-%, besonders bevorzugt 1 bis 5 Gew.-%, bezogen auf das absorbierende Polymer.

Vorzugsweise werden die Tenside im Lösungsmittel gelöst dosiert. Die Konzentration des Tensids in der Lösung beträgt typischerweise von 5 bis 80 Gew.-%, vorzugsweise 15 bis 60 Gew.-%, besonders bevorzugt 20 bis 55 Gew.-%.

Bei Verwendung von mehrwertigen Metallkationen werden typischerweise 0,01 bis 4 Gew.-%, vorzugsweise 0,05 bis 3 Gew.-%, besonders bevorzugt 0,1 bis 1 Gew.-%, des mehrwertigen Metallkations, bezögen auf das absorbierende Polymer, eingesetzt.

Die Gegenionen der mehrwertigen Metallionen unterliegen keiner Beschränkung, bei Verwendung eines Lösungsmittels sind Gegenionen bevorzugt, die eine ausreichende Löslichkeit gewährleisten, bevorzugt ist Sulfat. Vorzugsweise werden die Metallkationen als Lösung dosiert. Besonders bevorzugt als Lösungsmittel ist Wasser. Die Konzentration des mehrwertige Metallkations in der Lösung beträgt typischerweise von 1 bis 10 Gew.-%, vorzugsweise 2 bis 8 Gew.-%, besonders bevorzugt 3 bis 6 Gew.-%.

Die Reihenfolge, in der die Nachbehandlungsmittel dosiert werden unterliegt keiner Beschränkung, bevorzugt werden die mehrwertigen Metallkationen vor den Tensiden dosiert.

Die gelösten Nachbehandlungsmittel werden vorzugsweise auf das getrocknete wasserabsorbierende Polymer aufgesprüht und gemischt. Die Art des Mischens unterliegt keinen Beschränkungen, vorzugsweise werden Reaktionsmischer oder Misch- und Trocknungsanlagen, wie beispielsweise Lödige®-Mischer, BEPEX®-Mischer, NAUTP®-Mischer, SCHUGI®-Mischer, NARA®-Trockner und PROCESSALL®, verwendet. Überdies können auch Wirbelschichttrockner eingesetzt werden. Die Mischung wird zweckmäßigerweise mit einer Verweilzeit von 1 bis 180 Minuten, vorzugsweise von 2 bis 15 Minuten, und einer Drehzahl von 10 bis 1000 U/min, vorzugsweise von 50 bis 250 U/min, durchgeführt.

Nach dem letzten Schritt kann getrocknet werden. Die Trocknung kann im Mischer selbst erfolgen, durch Beheizung des Mantels oder Einblasen von Warmluft. Ebenso geeignet sind ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen im erfindungsgemäßen Verfahren liegen im Bereich 50 bis 250 °C, vorzugsweise bei 50 bis 200 °C, besonders bevorzugt bei 50 bis 180 °C. Die Verweilzeit bei dieser Temperatur im Reaktionsmischer oder Trockner beträgt zweckmäßigerweise unter 30 Minuten, vorzugweise unter 10 Minuten.

Die Trocknung wird vorzugsweise bei vermindertem Druck, vorzugsweise bei weniger als 500 mbar, besonders bevorzugt bei weniger als 200 mbar, durchgeführt und gegebenenfalls durch einen trockenen Gasstrom, vorzugsweise Stickstoff, in einer Menge von 20 bis 1000 l/kgh, vorzugsweise 100 bis 250 l/kgh, unterstützt.

Ein weiterer Gegenstand der Erfindung sind Hygieneartikel, welche die erfindungsgemäßen Superabsorber enthalten.

Die einsetzbaren absorbierenden Polymere sind insbesondere Polymere aus vernetzten (co)polymerisierten hydrophilen Monomeren, Polyasparaginsäure, Pfropf(co)polymere von einem oder mehreren hydrophilen Monomeren auf eine geeignete Pfropfgrundlage, vernetzte Cellulose- oder Stärkeether oder in wässrigen Flüssigkeiten quellbare Naturprodukte, wie beispielsweise Guarderivate. Bevorzugt handelt es sich bei dem zu vernetzenden Polymer um ein Polymer, das Struktureinheiten enthält, die sich von Acrylsäure oder deren Estern ableiten, oder die durch Pfropfcopolymerisation von Acrylsäure oder Acrylsäureestern auf eine wasserlösliche Polymermatrix erhalten wurden. Diese Hydrogele sind dem Fachmann bekannt und beispielsweise in der US-4,286,082, DE-C-27 06 135, US-A-4,340,706, DE-C-37 13 601, DE-C-28 40 010, DE-A-43 44 548, DE-A-40 20 780, DE-A-40 15 085, DE-A-39 17 846, DE-A-38 07 289, DE-A-35 33 337, DE-A-35 03 458, DE-A-42 44 548, DE-A-42 19 607, DE-A-40 21 847, DE-A-38 31 261, DE-A-35 11 086, DE-A-31 18 172, DE-A-30 28 043, DE-A-44 18 881, EP-A-0 801 483, EP-A-0 455 985, EP-A-0 467 073, EP-A-0 312 952, EP-A-0 205 874, EP-A-0 499 774, DE-A 26 12 846, DE-A-40 20 780, EP-A-0 205 674, US-A-5,145,906, EP-A-0 530 438, EP-A-0 670 073, US-A-4,057,521, US-A-4,062,817, US-A-4,525,527, US-A-4,295,987, US-A-5,011,892, US-A-4,076,663 oder US-A-4,931,497 beschrieben.

Zur Herstellung dieser absorbierenden Polymere geeignete hydrophile Monomere sind beispielsweise polymerisationsfähige Säuren, wie Acrylsäure, Methacrylsäure, Vinylsulfonsäure, Vinylphosphonsäure, Maleinsäure einschließlich deren Anhydrid, Fumarsäure, Itaconsäure, 2-Acrylamido-2-methylpropansulfonsäure, 2-Acrylamido-2-methylpropanphosphonsäure sowie deren Amide, Hydroxyalkylester und aminogruppen- oder ammoniumgruppenhaltige Ester und Amide sowie die Alkalimetall- und/oder Ammoniumsalze der Säuregruppen enthaltenden Monomeren. Des weiteren eignen sich wasserlösliche N-Vinylamide wie N-Vinylformamid oder auch Diallyldimethylammoniumchlorid. Bevorzugte hydrophile Monomere sind Verbindungen der allgemeinen Formel II

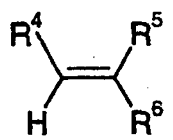

(II)

worin

$R^4$ Wasserstoff, Methyl, Ethyl oder Carboxyl,

$R^5$ $-COOR^7$, Hydroxysulfonyl oder Phosphonyl, eine mit einem $C_1$-$C_4$-Alkanol veresterte Phosphonylgruppe oder eine Gruppe der Formel III

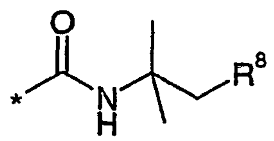

(III)

$R^6$ Wasserstoff, Methyl oder Ethyl,

$R^7$ Wasserstoff, $C_1$-$C_4$-Aminoalkyl, $C_1$-$C_4$-Hydroxyalkyl, Alkalimetall- oder Ammoniumion und

$R^8$ eine Sulfonylgruppe, eine Phosphonylgruppe oder eine Carboxylgruppe oder jeweils deren Alkalimetall- oder Ammoniumsalze, bedeuten.

Beispiele für $C_1$-$C_4$-Alkanole sind Methanol, Ethanol, n-Propanol, Isopropanol oder n-Butanol.

Besonders bevorzugte hydrophile Monomere sind Acrylsäure und Methacrylsäure, sowie deren Alkalimetall- oder Ammoniumsalze, beispielsweise Natriumacrylat, Kaliumacrylat oder Ammoniumacrylat.

Geeignete Pfropfgrundlagen für absorbierende Polymere, die durch Pfropfcopolymerisation olefinisch ungesättigter Säuren oder ihrer Alkalimetall- oder Ammoniumsalze erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Geeignete Polyalkylenoxide haben beispielsweise die Formel IV

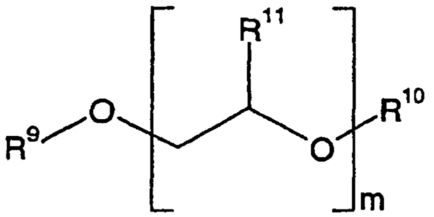

(IV)

worin

$R^9$, $R^{10}$ unabhängig voneinander Wasserstoff, Alkyl, Alkenyl oder Aryl,

$R^{11}$ Wasserstoff oder Methyl und

m eine ganze Zahl von 1 bis 10000 bedeuten.

$R^9$ und $R^{10}$ bedeuten bevorzugt Wasserstoff, $C_1$-$C_4$-Alkyl, $C_2$-$C_6$-Alkenyl oder Phenyl.

Bevorzugte absorbierende Polymere sind insbesondere Polyacrylate, Polymethacrylate sowie die In der US-4,931,497, US-5,011,892 und US-5,041,496 beschriebenen Pfropfpolymere.

Die absorbierenden Polymere sind bevorzugt vernetzt, d.h. sie enthalten Verbindungen mit mindestens zwei Doppelbindungen, die in das Polymernetzwerk einpolymerisiert sind. Geeignete Vernetzer sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Weiterhin einsetzbar im erfindungsgemäßen Verfahren sind auch Hydrogele, die unter Verwendung von Polyallylethern als Vernetzer und durch saure Homopolymerisation von Acrylsäure hergestellt werden. Geeignete Vernetzer sind Pentaerythritoltri- und -tetraallylether, Polyethylenglykoldiallylether, Ethylenglykoldiallylether, Glyceroldi- und Triallylether, Polyallylether auf Basis Sorbitol, sowie ethoxilierte Varianten davon.

Die bevorzugten Herstellverfahren für das einsetzbare Grundpolymer werden in "Modern Superabsorbent Polymer Technology", F.L. Buchholz and A.T. Graham, Wiley-VCH, 1998, Seiten 77 bis 84 beschrieben. Besonders bevorzugt sind Basispolymere, die im Kneter, wie beispielsweise in WO-A-01/38402 beschrieben, oder auf einem Bandreaktor, wie beispielsweise in EP-A-0 955 086 beschrieben, hergestellt werden.

Das absorbierende Polymer ist bevorzugt eine polymere Acrylsäure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere in Mengen von 0,001 bis 10 mol-%, vorzugsweise 0,01 bis 1 mol-% enthalten, ganz besonders bevorzugt sind jedoch Polymere, die durch radikalisch Polymerisation erhalten wurden und bei denen ein mehrfunktioneller ethylenisch ungesättigter Radikalvernetzer verwendet wurde, der zusätzlich noch mindestens eine freie Hydroxylgruppe trägt (wie beispielsweise Pentaerythritoltriallylether oder Trimethylolpropandiallylether).

Die absorbierenden Polymere können durch an sich bekannte Polymerisationsverfahren hergestellt werden. Bevorzugt ist die Polymerisation in wässriger Lösung nach dem Verfahren der sogenannten Gelpolymerisation. Dabei werden beispielsweise 15 bis 50 gew.-%ige wässrige Lösungen eines oder mehrerer hydrophiler Monomere und gegebenenfalls einer geeigneten Pfropfgrundlage in Gegenwart eines Radikalinitiatörs, bevorzugt ohne mechanische Durchmischung unter Ausnutzung des Trommsdorff-Norrish-Effektes (Makromol. Chem. 1, 169 (1947)), polymerisiert. Die Polymerisationsreaktion kann im Temperaturbereich zwischen 0 und 150°C, vorzugsweise zwischen 10 und 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden. zur Auslösung der Polymerisation können energiereiche elektromagnetische Strahlen oder die üblichen chemischen Polymerisationsinitiatoren herangezogen werden, beispielsweise organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxoverbindungen wie $(NH_4)_2S_2O_8$ oder $K_2S_2O_8$ oder $H_2O_2$. Sie können gegebenenfalls in Kombination mit Reduktionsmitteln wie Natriumhydrogensulfit und Eisen(II)-sulfat oder Redoxsystemen, welche als reduzierende Komponente eine aliphatische und aromatische Sulfinsäure, wie Benzolsulfinsäure und Toluolsulfinsäure oder Derivate dieser Säuren enthalten, wie Mannichaddukte aus Sulfinsäuren, Aldehyden und Aminoverbindungen, wie sie in der DE-A-13 01 566 beschrieben sind, verwendet werden. Durch mehrstündiges Nachheizen der Polymergele im Temperaturbereich 50 bis 130°C, vorzugsweise 70 bis 100°C, können die Qualitätseigenschaften der Polymere noch verbessert werden.

Die erhaltenen Hydrogele werden beispielsweise zu 0 bis 100 mol-%, bevorzugt 25 bis 100 mol-% und besonders bevorzugt zu 50 bis 85 mol-%, bezogen auf eingesetztes Monomer neutralisiert, wobei die üblichen Neutralisationsmittel verwendet werden können, bevorzugt Alkalimetallhydroxide oder-oxide, besonders bevorzugt jedoch Natriumhydroxid, Natriumcarbonat und Natriumhydrogencarbonat.

Üblicherweise wird die Neutralisation durch Einmischung des Neutralisationsmittels als wässrige Lösung oder bevorzugt auch als Feststoff erreicht. Das Gel wird hierzu mechanisch zerkleinert, beispielsweise mittels eines Fleischwolfes und das Neutralisationsmittel wird aufgesprüht, übergestreut oder aufgegossen, und dann sorgfältig untergemischt. Dazu kann die erhaltene Gelmasse noch mehrmals zur Homogenisierung gewolft werden. Die neutralisierte Gelmasse wird dann mit einem Band- oder Walzentrockner getrocknet bis der Restfeuchtegehalt vorzugsweise unter 10 Gew.-%, insbesondere unter 5 Gew.-% liegt. Das getrocknete Hydrogel wird hiernach gemahlen und gesiebt, wobei zur Mahlung üblicherweise Walzenstühle, Stiftmühlen oder Schwingmühlen eingesetzt werden können. Die Partikelgröße des gesiebten Hydrogels beträgt üblicherweise unter 1000 μm, häufig unter 700 μm, vorzugsweises unter 500 μm, besonders bevorzugt unter 400 μm, ganz besonders bevorzugt unter 300 μm, wobei der Anteil an Partikeln mit kleiner 10 μm unter 1 Gew.-% beträgt. Ganz besonders bevorzugt ist er Siebschnitt 45 bis 250 μm.

Kleinere Partikelgrößen führen insbesondere zu einer höheren Blutabsorption.

Die Nachvernetzung wird üblicherweise so durchgeführt, dass eine Lösung des Oberflächennachvernetzers auf das Hydrogel oder das trockene Grundpolymerpulver aufgesprüht wird. Im Anschluss an das Aufsprühen wird das Polymerpulver thermisch getrocknet, wobei die Vernetzungsreaktion sowohl vor als auch während der Trocknung stattfinden kann.

Bevorzugt ist das Aufsprühen einer Lösung des Vernetzers in Reaktionsmischern oder Misch- und Trocknungsanlagen, wie beispielsweise Lödige®-Mischer, BEPEX®-Mischer, NAUTA®-Mischer, SCHUGI®-Mischer, NARA®-Trockner und PROCES-SALL®. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen liegen im Bereich 50 bis 250 °C, bevorzugt bei 50 bis 200 °C, und besonders bevorzugt bei 50 bis 150 °C. Die bevorzugte Verweilzeit bei dieser Temperatur im Reaktionsmischer oder Trockner beträgt unter 30 Minuten, besonders bevorzugt unter 10 Minuten.

Der Vernetzer wird bevorzugt in nicht selbst-reaktiven Lösemitteln gelöst, bevorzugt in niederen Alkoholen, wie beispielsweise Methanol, Ethanol, Propandiol, Methylenglykol, ganz besonders bevorzugt in wässrigen Lösungen solcher geeigneter Alkohole, wobei der Alkoholgehalt der Lösung 10 bis 90 Gew.-%, besonders bevorzugt 40 bis 60 Gew.-% beträgt.

Der Vernetzer wird dabei in einer Menge von 0,01 bis 1 Gew.-%, bezogen auf das eingesetzte Polymer, verwendet, und die Vernetzerlösung selbst in einer Menge von 1 bis 20 Gew.-%, bevorzugt 5 bis 15 Gew.-%, bezogen auf das eingesetzte Polymer, verwendet.

Nicht nachvernetzte hydrogelbildende Polymere (Grundpolymere) sind besonders geeignet. Bevorzugt werden daher Mischungen, die einen hohen Anteil an Grundpolymer aufweisen, oder reines Grundpolymer eingesetzt. Der Anteil nicht nachvernetzter Pölymerpartikel in der Mischung beträgt typischerweise mindestens 20 Gew.-%, vorzugsweise mindestens 50 Gew.-%, besonders bevorzugt mindestens 70 Gew.-%.

Die erfindungsgemäßen Flüssigkeiten absorbierende Polymerpartikel eignen sich besonders zur Absorption

von Blut und/oder Körperflüssigkeiten in Hygieneartikeln, wie beilspielsweise Inkontinenzartikeln, Binden, Tampons, Einlagen. Dazu können die erfindungsgemäßen Polymerpartikel mit Fasern, wie beispielsweise Zellulose, sowie Faservlies zu absorbierenden Verbundstoffen verarbeitet werden.

Selbstverständlich können den Polymerpartikeln bei Bedarf weitere Stoffe, wie Bakterizide, Biozide, Duftstoffe, Stabilisatoren, Farbstoffe, Indiktoren, Entschäumer, Komplexbildner, Netzmittel, Verdickungsmittel, Dispersionen, Plastifizierungsmittel, Retentionsmittel, Pigmente, Füllstoffe und weitere dem Fachmann bekannte Hilfsmittel zugesetzt werden.

Geeignete Füllstoffe sind beispielsweise Sand oder Tonminerale, wie sie in der WO=A-04/018005 auf den Seiten 16 bis 20 beschrieben werden. üblicherweise weisen die Füllstoffe Partikelgrößen von 10 bis 1.000 µm, vorzugsweise 150 bis 850 µm, besonders bevorzugt 300 bis 600 µm, auf. Die Partikelgrößen lassen sich nach üblichen Methoden über entsprechende Siebfräktionen einstellen. Vorzugsweise haben die erfindungsgemäßen Polymerpartikel und die Füllstoffe ähnliche Partikelgrößen, wodurch eine Entmischung verhindert wird.

Darüber hinaus können beispielsweise strahlungs- und/oder wärmehärtbaren Materialien (Vernetzer) und/oder Hydrophobierungsmittel zugesetzt werden. Geeignete Hydrophobierungsmittel sind übliche wässrige Paraffindispersionen oder Silikone.

Zur Bestimmung der Güte der Nachbehandlung werden die erfindungsgemäßen Polymerpartikel mit den Testmethoden geprüft, die nachfolgend beschrieben sind.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Das quellbare hydrogelbildende Polymer wird vor der Messung gut durchmischt.

Blutabsorption (BA):

Diese Methode dient zur Bestimmung der Blutabsorption absorbierender Polymere innehalb von 30 Minuten.

Versuchsaufbau:

- Kunststoffbehälter, rund, Innendurchmesser 50 ± 0,2 mm, Innenhöhe 20 ± 0,2 mm (Behälter I)
- Kunststoffzylinder mit Netzteil (400 mesh = 36 µm große Löcher), Innendurchmesser 25 ± 0,2 mm, Höhe 40 ± 0,2 mm (Behälter II)
- Petrischale mit Deckel, Durchmesser 140 mm, Höhe 75 mm
- Stoppuhr
- Analysenwaage mit einer Genauigkeit von ± 0,0001 g.
- Defibriniertes Schafsblut der Firma Oxoid GmbH, D-46467 Wesel

Versuchsdurchführung:

0,2 g absorbierendes Polymer werden in den Behälter II, dessen Leergewicht vorher bestimmt wurde, eingewogen. Behälter I wird mit 15 g defibriertem Schafsblut beschickt. Behälter II wird nun in Behälter I gestellt, dieser Aufbau in die Petri-Schale gestellt, die Petri-Schale durch den Deckel verschlossen und die Zeitmessung gestartet. Nach einer Zeitvon 30 Minuten wird Behälter II aus Behälter I genommen, die äußere Seite des Behälters mit einem Tuch gereinigt und das Gewicht des Behälters II anschließend bestimmt. Aus der Differenz dieses Gewichtes und des Leergewichtes von Behälter II sowie der eingesetzten Polymer-Masse (0,2 g) wird die absorbierte Blutmenge und daraus die Blutabsorption berechnet.

Berechnung:

$$\frac{\text{Absorption an Blut [g]}}{\text{Einwaage absorbierendes Polymer [g]}} = \text{Blutabsorption [g/g]}$$

Der Test wurde mit dem feuchten bzw. getrockneten absorbierenden Polymers durchgeführt.

Zentrifugenretentionskapazität (CRC)

Bei dieser Methode wird die freie Quellbarkeit des absorbierenden Polymers im Teebeutel bestimmt. Zur Bestimmung der CRC werden 0,2000 ± 0,0050 g absorbierendes Polymer in einem 60 x 85 mm großen Teebeutel eingewogen, der anschließend verschweißt wird. Der Teebeutel wird für 30 Minuten in einen Überschuss von 0,9 gew.-%iger Kochsalzlösung gegeben (mindestens 0,83 l Kochsalzlösung/1 g Polymerpulver). Anschließend wird der Teebeutel 3 Minuten lang bei 250 G zentrifugiert. Die Bestimmung der vom Hydrogel festgehaltenen Flüssigkeitsmenge geschieht durch Auswägen des zentrifugierten Teebeutels.

Die Zentrifugenretentionskapazität kann auch nach der von der EDANA (European Disposables and Nonwovens Association) empfohlenen Testmethode Nr. 441.2-02 "Centrifuge retention capacity" bestimmt werden.

Retention (FSC):

Bei dieser Methode wird ebenfalls die freie Quellbarkeit des absorbierenden Polymers im Teebeutel bestimmt. Die Methode wird analog zur Bestimmung der Zentrifugenretentionskapazität durchgeführt, mit der Ausnahme, dass der Teebeutel nicht zentrifugiert wird. Stattdessen wird der Teebeutel für 10 Minuten abtropfen gelassen, indem er an einer Ecke aufgehängt wird.

Die Retention kann auch nach der von der EDANA (European Disposables and Nonwovens Association) empfohlenen Testmethode Nr. 440.2-02 "Free swell capacity" bestimmt werden.

Beispiele

Beispiele 1 bis 10:

In einem Lödige®-Pflugscharmischer vom Typ M5/20 Laborchargenmischer wurden 1 kg kommerziell verfügbares wässrige Flüssigkeiten absorbierendes Polymer (Hysorb F) vorgelegt und die angegebene Menge an Tensid-Lösung, bezogen auf das absorbierende Polymer, als 50 gew.-%ige Lösung in Diethylenglykolmonobutylether aufgesprüht und 10 Minuten nachgemischt.

ggf. die angegebene Menge an Aluminiumsulfat-Lösung, bezogen auf das absorbierende Polymer, als 26,8 gew.%ige wässrige Lösung aufgesprüht und 10 Minuten nachgemischt und

Die Drehzahl des Mischers betrug 125 U/min.

In den Beispielen, in denen die wässrige Aluminiumsulfatlösung aufgesprüht wurde, wurde zusätzlich getrocknet. Getrocknet wurde bei 70°C, einem Druck von 150 mbar und einer Verweilzeit von 16 Stunden. Die Trocknung wurde durch eine Gasstrom von 200 l/h Stickstoff unterstützt.

Die Ergebnisse sind in den nachfolgenden Tabellen zusammengestellt.

Tabelle 1: Zusatz von Kationenlösungen

| Beispiel | Tensid-Lösung | Kationen-Lösung | BA, feucht [g/g] | BA, trocken [g/g] | FSC, feucht [g/g] | FSC, trocken [g/g] | CRC, feucht [g/g] | CRC, trocken [g/g] |
|---|---|---|---|---|---|---|---|---|
| 1 | 5% Lutensol® XP 30 | | 18,2 | 20,1 | 33,1 | 36,1 | 25,5 | 27,6 |
| 2 | 5% Lutensol® XP 30 | 4% $Al_2(SO_4)_3$ | 25,8 | 29,0 | 43,2 | 47,2 | 19,2 | 22,9 |
| 3 | 5% Lutensol® XL 30 | | 27,7 | 20,1 | 34,5 | 38,1 | 26,4 | 29,6 |

(fortgesetzt)

| Beispiel | Tensid-Lösung | Kationen-Lösung | BA, feucht [g/g] | BA, trocken [g/g] | FSC, feucht [g/g] | FSC, trocken [g/g] | CRC, feucht [g/g] | CRC, trocken [g/g] |
|---|---|---|---|---|---|---|---|---|
| 4 | 5% Lutensol® XL 30 | 4% $Al_2(SO_4)_3$ | 29,6 | 31,2 | 45,5 | 44,7 | 19,8 | 23,5 |
| Hysorb® F: superabsorbierendes Polymer (BASF Aktiengesellschaft, DE)<br>Lutensol® XP 30: ethoxilierter $C_{10}$-Alkohol (BASF Aktiengesellschaft, DE)<br>Lutensol® XL 30: ethoxilierter ungesättigter $C_{10}$-Alkohol (BASF Aktiengesellschaft, DE) | | | | | | | | |

Tabelle 2: Konzentrationsabhängigkeit

| Beispiel | Tensid-Lösung | Kationen-Lösung | BA, feucht [g/g] | FSC, trocken [g/g] | CRC, trocken [g/g] |
|---|---|---|---|---|---|
| 5 | 0,5% Lutensol® XL 40 | | 11,8 | 36,3 | 27,5 |
| 6 | 1% Lutensol® XL 40 | | 20,5 | 36,3 | 27,0 |
| 7 | 5% Lutensol® XL 40 | | 21,8 | 36,6 | 27,6 |
| 8 | 0,5% Lutensol® TO 3 | | 12,7 | 38,0 | 29,5 |
| 9 | 1% Lutensol® TO 3 | | 16,3 | 37,0 | 29,6 |
| 10 | 5% Lutensol® TO 3 | | 20,1 | 36,5 | 29,7 |
| Lutensol® XL 40: ethoxilierter ungesättigter $C_{10}$-Alkohol (BASF Aktiengesellschaft, DE)<br>Lutensol® TO 3: ethoxilierter iso-$C_{13}$-Alkohol (BASF Aktiengesellschaft, DE) | | | | | |

Beispiele 11 bis 16

Die Versuche wurden analog zu den Beispielen 1 bis 10 durchgeführt. Die angegebene Menge an Tensid-Lösung, bezogen auf das absorbierende Polymer, wurde als 25 gew.-%ige Lösung aufgesprüht

Tabelle 3: Lösungsmitteleinfluss

| Beispiel | Tensid-Lösung | Lösungsmittel | BA, feucht [g/g] | FSC, trocken [g/g] | CRC, trocken [g/g] |
|---|---|---|---|---|---|
| 11 (Vgl.) | 0,25% Tween® 20 | Wasser | 8,8 | 40,0 | 30,7 |
| 12 (Vgl.) | 0,5% Tween® 20 | Wasser | 5,2 | 39,0 | 30,4 |
| 13 (Vgl.) | 1% Tween® 20 | Wasser | 9,1 | 40,1 | 27,7 |
| 14 (Vgl.) | 5% Tween® 20 | Wasser | 7,6 | 43,3 | 26,4 |
| 15 | 1,25% Tween® 20 | DEGMBE | 17,7 | 21,9 | 16,9 |
| 16 | 2,5% Tween® 20 | DEGMBE | 19,5 | 21,4 | 16,5 |
| Tween® 20: ethoxiliertes Sorbitanmonolaurat (ICI Ltd., UK)<br>DEGMBE: Diethylenglykolmonobutylether | | | | | |

**Patentansprüche**

**1.** Blut und/oder Körperflüssigkeiten absorbierende Polymerpartikel, enthaltend

a) mindestens ein einpolymerisiertes ethylenisch ungesättigtes, säuregruppentragendes Monomer,
b) mindestens einen einpolymerisierten Vernetzer,
c) gegebenenfalls ein oder mehrere mit a) copolymerisierbare einpolymerisierte ethylenisch und/oder allylisch ungesättigte Monomere,
d) gegebenenfalls ein oder mehrere wasserlösliche Polymere, auf die die Monomere a), b) und ggf. c) zumindest

teilweise aufgepfropft sind, und
e) gegebenenfalls ein oder mehrere umgesetzte Nachvernetzer,

wobei die Polymerpartikel mit mindestens einem Tensid und mindestens einem Lösungsmittel der allgemeinen Formel I

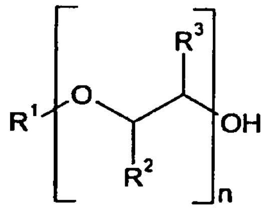

(I)

in der

$R^1$ $C_1$-$C_8$-Alkyl, wobei der Rest halogensubstituiert sein kann,
$R^2$, $R^3$ unabhängig voneinander Wasserstoff oder Methyl und
n eine ganze Zahl von 0 bis 5 bedeuten,

beschichtet sind und die trockenen Polymerpartikel eine Blutabsorption von mindestens 15 g/g aufweisen.

**2.** Polymerpartikel gemäß Anspruch 1, wobei das Tensid ein nichtionisches Tensid mit einem HLB-Wert von 2 bis 18 ist.

**3.** Polymerpartikel gemäß einem der Ansprüche 1 oder 2, wobei das Lösungsmittel eine Verbindung der allgemeinen Formel I ist, in der

$R^1$ $C_2$-$C_6$-Alkyl,
$R^2$, $R^3$ Wasserstoff und
n eine ganze Zahl von 1 bis 3

bedeuten.

**4.** Polymerpartikel gemäß einem der Ansprüche 1 bis 3, wobei die Polymerpartikel zusätzlich mit mindestens einem mehrwertigen Metallkation beschichtet sind.

**5.** Polymerpartikel gemäß einem der Ansprüche 1 bis 4, wobei das mehrwertige Metallkation ein Aluminiumkation ist.

**6.** Polymerpartikel gemäß einem der Ansprüche 1 bis 5, wobei die Polymerpartikel nicht nachvemetzt sind.

**7.** Mischung von Polymerpartikeln gemäß einem der Ansprüche 1 bis 5, wobei mindestens 20 Gew.-% der Polymerpartikel nicht nachvernetzt sind.

**8.** Verfahren zur Herstellung von Hygieneartikeln, **dadurch gekennzeichnet, dass** man Polymerpartikel gemäß einem der Ansprüche 1 bis 7 als Absorbens einsetzt.

**9.** Hygieneartikel enthaltend Polymerpartikel gemäß einem der Ansprüche 1 bis 7.

## Claims

**1.** Polymeric particles capable of absorbing blood and/or body fluids and comprising

a) at least one interpolymerized ethylenically unsaturated acid-functional monomer,
b) at least one interpolymerized crosslinker,
c) optionally one or more interpolymerized ethylenically and/or allylically unsaturated monomers copolymerizable with a),
d) optionally one or more water-soluble polymers onto which said monomers a), b) and optionally c) are at least

partially grafted, and
e) optionally one or more reacted postcrosslinkers,

wherein said polymeric particles are coated with at least one surfactant and with at least one solvent of the general formula I

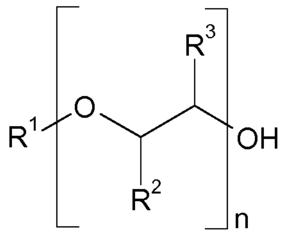

(I)

where

$R^1$ is $C_1$-$C_8$-alkyl with or without halogen substitution,
$R^2$, $R^3$ are independently hydrogen or methyl, and
n is an integer from 0 to 5,

and the polymeric particles have a blood absorbence of at least 15 g/g in the dry state.

**2.** The polymeric particles according to claim 1 wherein the surfactant is a nonionic surfactant having an HLB value in the range from 2 to 18.

**3.** The polymeric particles according to claim 1 or claim 2 wherein the solvent is a compound of the general formula I where

$R^1$ is $C_2$-$C_6$-alkyl,
$R^2$, $R^3$ are each hydrogen, and
n is an integer from 1 to 3.

**4.** The polymeric particles according to any of claims 1 to 3 that are further coated with at least one multivalent metal cation.

**5.** The polymeric particles according to any of claims 1 to 4 wherein the multivalent metal cation is an aluminum cation.

**6.** The polymeric particles according to any of claims 1 to 5 that are not postcrosslinked.

**7.** A mixture of polymeric particles according to any of claims 1 to 5 wherein not less than 20% by weight of said polymeric particles are not postcrosslinked.

**8.** A process for producing a hygiene article, which comprises utilizing polymeric particles according to any of claims 1 to 7 as an absorbent.

**9.** A hygiene article comprising polymeric particles according to any of claims 1 to 7.

## Revendications

**1.** Particules polymères absorbant le sang et/ou les liquides corporels contenant :

a) au moins un monomère polymérisé et éthyléniquement insaturé portant des groupes acides,
b) au moins un agent de réticulation polymérisé,
c) éventuellement un ou plusieurs monomères éthyléniquement et/ou allyliquement insaturés polymérisés et copolymérisables avec a),
d) éventuellement un ou plusieurs polymères solubles dans l'eau sur lesquels les monomères a), b) et éventuellement c) sont au moins partiellement greffés, et
e) éventuellement un ou plusieurs agents de post-réticulation réagis,

les particules polymères étant enrobées d'au moins un tensioactif et d'au moins un solvant de formule générale I:

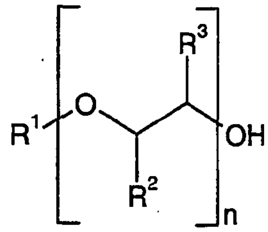

(I)

dans laquelle :

$R^1$ représente un alkyle en $C_1$-$C_8$, le radical pouvant être substitué par un halogène,
$R^2$, $R^3$ représentent indépendamment l'un de l'autre l'hydrogène ou un méthyle, et
n signifie un nombre entier de 0 à 5,

et les particules polymères sèches présentant une absorption du sang d'au moins 15 g/g.

**2.** Particules polymères selon la revendication 1, dans lesquelles le tensioactif est un tensioactif non ionique avec une valeur HLB de 2 à 18.

**3.** Particules polymères selon l'une des revendications 1 ou 2, dans lesquelles le solvant est un composé de formule générale I, dans laquelle :

$R^1$ signifie un alkyle en $C_2$-$C_6$,
$R^2$, $R^3$ signifient un hydrogène, et
n signifie un nombre entier de 1 à 3.

**4.** Particules polymères selon l'une des revendications 1 à 3, dans lesquelles les particules polymères sont en plus enrobées d'au moins un cation de métal polyvalent.

**5.** Particules polymères selon l'une des revendications 1 à 4, dans lesquelles le cation de métal polyvalent est un cation d'aluminium.

**6.** Particules polymères selon l'une des revendications 1 à 5, dans lesquelles les particules polymères sont post-réticulées.

**7.** Mélange de particules polymères selon l'une des revendications 1 à 5, dans lequel au moins 20 % en poids des particules polymères ne sont pas post-réticulées.

**8.** Procédé de fabrication d'articles d'hygiène, **caractérisé en ce qu'**on utilise comme produit d'absorption les particules polymères selon l'une des revendications 1 à 7.

**9.** Articles d'hygiène contenant les particules polymères selon l'une des revendications 1 à 7.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 9955767 A
- DE 19909653 A
- WO 0010496 A
- EP 0759460 A
- WO 9519191 A
- JP 6345980 A
- US 4286082 A
- DE 2706135 C
- US 4340706 A
- DE 3713601 C
- DE 2840010 C
- DE 4344548 A
- DE 4020780 A
- DE 4015085 A
- DE 3917846 A
- DE 3807289 A
- DE 3533337 A
- DE 3503458 A
- DE 4244548 A
- DE 4219607 A
- DE 4021847 A
- DE 3831261 A
- DE 3511086 A
- DE 3118172 A
- DE 3028043 A
- DE 4418881 A
- EP 0801483 A
- EP 0455985 A
- EP 0467073 A
- EP 0312952 A
- EP 0205874 A
- EP 0499774 A
- DE 2612846 A
- EP 0205674 A
- US 5145906 A
- EP 0530438 A
- EP 0670073 A
- US 4057521 A
- US 4062817 A
- US 4525527 A
- US 4295987 A
- US 5011892 A
- US 4076663 A
- US 4931497 A
- US 5041496 A
- EP 0343427 A
- WO 0138402 A
- EP 0955086 A
- DE 1301566 A

### In der Beschreibung aufgeführte Nicht-Patentliteratur

- **F.L. BUCHHOLZ ; A.T. GRAHAM.** Modern Superabsorbent Polymer Technology. Wiley-VCH, 1998, 77-84
- *Makromol. Chem.,* 1947, vol. 1, 169